## Europäisches Patentamt

## European Patent Office

## Office européen des brevets

(11) Veröffentlichungsnummer: **0 019 622**

**B2**

(12) # NEUE EUROPÄISCHE PATENTSCHRIFT

(45) Veröffentlichungstag der neuen Patentschrift:
12.10.88

(21) Anmeldenummer: 79901040.0

(22) Anmeldetag: 20.08.79

(86) Internationale Anmeldenummer:
PCT/DE 79/00089

(87) Internationale Veröffentlichungsnummer:
WO 80/00413 (20.03.80 Gazette 80/06)

(51) Int. Cl.⁴: **A 61 L 2/16**, A 61 L 2/10,
A 61 L 2/02, A 61 L 2/00,
A 61 G 15/00, A 61 B 19/00

(54) **VERFAHREN UND VORRICHTUNG ZUM STERILHALTEN CHIRURGISCHER ODER ZAHNÄRZTLICHER HANDSTÜCKE.**

(30) Priorität: 21.08.78 DE 2836532

(43) Veröffentlichungstag der Anmeldung:
10.12.80 Patentblatt 80/25

(45) Bekanntmachung des Hinweises auf die Patenterteilung:
28.11.84 Patentblatt 84/48

(45) Bekanntmachung des Hinweises auf die Entscheidung über den Einspruch:
12.10.88 Patentblatt 88/41

(84) Benannte Vertragsstaaten:
AT CH FR GB LU NL SE

(56) Entgegenhaltungen:
EP-A-0 000 773
EP-A-0 001 285
DE-A-2 209 961
DE-A-2 311 504
DE-A-2 614 776
DE-A-2 805 934
DE-B-1 032 894
GB-A-1 498 740
GB-A-1 512 146
US-A-2 235 296

Pschyrembel, Klin. Wörterbuch 1977, S. 240-241

Die Akte enthält technische Angaben, die nach dem Eingang der Anmeldung eingereicht wurden und die nicht in dieser Patentschrift enthalten sind.

(73) Patentinhaber: Scheicher, Hans, Dr.med. Dr.med.dent., Rondell Neuwittelsbach 4, D-8000 München 19 (DE)

(72) Erfinder: Scheicher, Hans, Dr.med. Dr.med.dent., Rondell Neuwittelsbach 4, D-8000 München 19 (DE)

(74) Vertreter: Weisert, Annekäte, Dipl.- Ing. Dr.- Ing., Patentanwälte Kraus Weisert & Partner Thomas-Wimmer- Ring 15, D-8000 München 22 (DE)

## Beschreibung

Die Erfindung betrifft eine Vorrichtung zum Sterilhalten chirurgischer oder zahnärztlicher Handstücke, aus denen Fluide für Antriebs- und/oder Kühl- und/oder Spülzwecke in eine Körperhöhle des Patienten austreten, bei der das zumindest eine Fluid zwischen seiner Quelle oder seinem Vorratsbehälter und der Austrittsöffnung im Handstück ersten entkeimenden Mitteln ausgesetzt wird, welche als Keimsperre zwischen Austrittsöffnung und Quelle oder Vorratsbehälter wirken, und das zumindest eine Fluid im Bereich seiner Quelle oder seines Vorratsbehälters zweiten entkeimenden Mitteln ausgesetzt wird, wobei eine Dosiervorrichtung für ein entkeimendes Mittel vorgesehen ist.

An zahnärztlichen und chirurgischen Arbeitsplätzen werden verschiedene Handstücke verwendet, mit denen im Mund des Patienten oder in Wundhölen gearbeitet wird. Dies sind insbesondere sogenannte Luftturbinen, Handmotore, Spritz- bzw. Blashandstücke und sogenannte Zahnsteinentferner.

Die Luftturbinen werden nach dem Prinzip der Wasserräder, jedoch mit Druckluft, angetrieben. Beidseitig des Turbinenrades ist je ein Lager angebracht. Wegen der hohen Drehzahlen können diese Lager nicht angedichtet werden.

Außerhalb der Lager, die sich im Kopfstück, im vordersten Teil des Bohrkopfes befinden, sind Öffnungen vorhanden.

Durch die eine Öffnung ragt das Fräs- oder Schleifinstrument, die an der Gegenseite liegende Öffnung wird zum Wechseln des Fräs- oder Schleifinstrumentes benutzt. Durch diese Öffnungen gelangt Druckluft aus dem Bohrkopf in den Mund des Patienten. Ferner sind solche Luftturbinen mit Öffnungen am vordersten Teil des Bohrkopfes ausgestattet, aus denen Kühlwasser auf den Fräser oder Schleifer gespritzt wird. Dies ist wegen der hohen Drehzahlen und der damit verbundenen Erwärmung notwendig, um keine Schädigung am Zahn oder Knochen hervorzurufen.

Es gibt ferner mit Druckluft als auch elektrisch betriebene Handmotore, auf die Bohrköpfe aufsetzbar sind. Der Bohrer wird hier über Getriebe angetrieben. Auch diese Handstücke haben am Bohrkopf Kühlwasserausgänge zur Kühlung der Bohrer.

Spritz- und Blashandstücke haben die Aufgabe, flüssige Kühl- und/oder Spülfluide an die zu versorgende Stelle zu bringen oder Blasluft, z. B. zum Trockenblasen der Kavitäten.

Die Zahnsteinentfernungshandstücke haben ebenfalls Kühlwasserausgänge am vordersten Kopfteil.

Alle beschriebenen Handstücke werden über Zuführungsleitungen oder Schläuche mit den jeweiligen Fluiden z. B. Druckluft und Wasser versorgt. Diese Fluide sind normalerweise nicht keimfrei.

Zum anderen wird bei vielen dieser Handstücke ein Nachtropfen durch sogenannte Rücksaugsysteme verhindert, die automatisch beim Abschalten zumindest die Flussigkeit unmittelbar vor und in den Leitungen im Bohrkopf in die Zuführungsleitungen oder Schläuche zurückziehen Hierdurch werden Keime, die vom Mund des Patienten an die Handstücke gelangen, in diese eingesaugt.

Insbesondere das in den Zuführungsleitungen und den Vorratsbehältern in den Behandlungspausen stehende und meist zusätzlich noch erwärmte Fluid bildet einen fast idealen Nährboden für die Vermehrung der Keime, so daß es sogar vorgekommen ist, daß die Zuführungsleitungen von Kulturen der Keime soweit zugesetzt wurden, daß die Fluidzufuhr zu den Austrittsöffnungen eingeschränkt oder unterbrochen wurden.

An eine Sterilität im Wundbereich ist daher auch dann nicht mehr zu denken, wenn die Handstücke, soweit dies aufgrund ihrer Materialien möglich ist, von Zeit zu Zeit sterilisiert oder mit einer keimtötenden Flüssigkeit abgewischt werden. Hieraus resultiert eine unvermeidliche Infektionsgefahr im Wundgebiet.

Aus der GB-A-1 498 740 ist eine Vorrichtung zum Sterilhalten chirurgischer oder zahnärztlicher Handstücke, aus denen Fluide für Antriebs- und/oder Kühl- und/oder spülzwecke in eine Körperhöhle des Patienten austreten, bei dem das zumindest eine Fluid zwischen seiner Quelle oder seinem Vorratsbehälter und der Austrittsöffnung im Handstück ersten entkeimenden Mitteln ausgesetzt wird, welche die Keime während des Durchfließens des Fluides abtöten, bekannt, bei dem eine Metallionen emittierende Quelle, wie beispielsweise ein Kupferdraht, im Inneren der zu einem Handstück führenden Leitung vorgesehen ist. Die Verwendung einer solchen Quelle bewirkt nur dann eine ausreichende Keimabtötung, wenn die Konzentration der abgegebenen Metallionen sehr groß ist. Da das durch die Leitung geführte Spülwasser direkt in den Körper- und Wundbereich gerät, dürften bei ihm die Zugaben von derartigen Metallionen unter keinen Umständen höher sein als dies aus gesundheitlichen Gründen im Leitungswasser zulässig ist. Die danach zulässigen Dosierungen sind jedoch sehr gering, da die Metallionen in größerer Konzentration giftig sind. Aufgrund des intermittierenden Betriebs zahnärztlicher und chirurgischer Handstücke bleibt ein Teil der Flüssigkeit länger in der Leitung, so daß die Abgabe der Metallionen auf so geringe Konzentrationen eingestellt sein muß, daß auch in diesen Fällen-beispielsweise bei der Abschaltung über Nacht-die Dosierung nicht zu hoch wird. Dies bewirkt jodoch, daß bei einem kontinuierlichen Durchfluß von Spülflüssigkeit oder bei nur kurzfristig unterbrochenem Betrieb, bei dem jeweils Bakterien zurückgesaugt werden, die Abgabe der Metallionen nicht mehr ausreicht, um eine ausreichende Desinfektionswirkung zu erzielen.

Eine verbrauchsabhängige, genau dosierte Anreicherung der Spülflüssigkeit mit derartigen Metallionen in der zum Handstück geführten Leitung, in der pro Minute 0 bis 4.000 ml fließen, ist jedoch auf diesem Wege nicht möglich.

Dies bedeutet, daß im Falle eines hohen Durchflusses das aus dem Vorratsbehälter entnommene, im Normalfall nicht sterile Kühlfluid, wie beispielsweise Leitungswasser, nicht ausreichend entkeimt wird. Die Aussichten, daß aus dem Handstück zurückgesaugte Bakterien aufgrund der dann größeren Verweildauer in der Leitung abgetötet werden, sind zwar grösser. Wenn eine vollständige Entkeimung nicht stattfindet, können diese jedoch im Vorratsbehälter unter den dort für sie günstigen Temperaturbedingungen weiterwuchern, so daß es nach wie vor dazu kommen kann, daß die Behälter und Zuführungsleitungen ganz von Keimkulturen zugesetzt sind, bis die Fluidzufuhr zu den Austrittsöffnung eingeschränkt oder unterbrochen wird.

Außerdem ist eine Vorrichtung der eingangs genannten Art in der nachveröffentlichen EP-A-773 beschrieben, die jedoch keine wirksame Keimabtötung im Rahmen der von den gesundheitsbehördlichen Vorschriften zugelassenen Toleranzen bei sehr stark variierendem Verbrauch an Spiel- und Kühlflüssigkeit ermögicht.

Aufgabe der Erfindung ist es demgegenüber, eine Vorrichtung der eingangs ganannten Art so auszubilden, daß sie eine wirksame Keimabtötung im Rahmen der nach den gesundheitsbehördlichen Vorschriften zulässigen Toleranzen für die beigegebenen Entkeimungsmittel ermöglicht, und zwar im Hinblick auf den bei zahnärztlichen und chirurgischen Behandlungselementen sehr start variierenden Verbrauch an Spülund Kühlflüssigkeit, wie es im Bereich von Nichtgebrauch der Vorrichtung über kurzzeitigen intermittierenden Betrieb bis zum kontinuierlichen Betrieb auftritt.

Diese Aufgabe wird erfindungsgemäß dadurch gelöst, daß die Dosiervorrichtung für ein zu entkeimendes Mittel ersten an eine Leitung über ein erstes Ventil anschließbaren Behälter, aus dem unten eine über ein zweites Ventil freigebbare Leitung herausführt und in den ein Injektor für die Freigabe einer dosierten Menge eines entkeimenden Mittels mündet, mit einem Schwimmer und einem Schwimmerventil, das mit den beiden Ventilen und dem Injektor derart zusammenwirkt, daß bei ausreichender Füllung des ersten Behälters das erste Ventil bei geschlossenem zweiten Ventil abschaltet und anschließend der Injektor erregt wird, und daß nach dem Entkeimen der im ersten Behälter befindlichen Wassermenge das zweite Ventil geöffnet wird, und daß die Dosiervorrichtung einen zweiten Behälter enthält, in den die Leitung mündet, an dem unten eine Leitung zu einem Verbraucher führt und der einen zweiten Schwimmer mit einem Schwimmerventil enthält, so daß bei vollem zweiten Behälter das entkeimte Verbrauchswasser über das Ventil mit definertem Ausfluß abgegeben wird.

Auf diese Weise wird eine trotz stark wechselndem Verbrauch an Spül- und Kühlflüssigkeit ausreichende Entkeimung innerhalb der zulässigen Toleranzen erreicht.

Es ist besonders zweckmäßig, wenn für das gasförmige und für das flüssige Fluid ein gemeinsamer Vorratsbehälter vorgesehen ist, der je einen Auslaß für jedes der Fluide aufweist. Das unter Druck in den Vorratsbehälter eingebrachte gasförmige Fluid, das in dem Behälter unter Überdruck steht, ersetzt zudem eine Druckpumpe für das flüssige Fluid, da dieses durch den wählbaren Druck des darüber befindlichen Gasraumes über die Zuführungsleitungen zu den Austrittsöffnungen im Handstück mit erwünschtem Druck gefördert wird. Um ein Mitreißen von flüssigen Fluidpartikeln in dem gasförmigen Fluid zu vermeiden, ist zweckmäßigerweise zumindest ein austauschbares und vorzugsweise keimtötendes Filter in der zumindest einen zwischen der Vorratsbehälter und den Austrittsöffnungen im Handstück verlaufenden Zuführungsleitung für das zumindest eine Fluid vorgesehen. Ein ähnliches Filter ist auch in der Zuführungsleitung für das andere Fluid zweckmäßig, um ein Verstopfen der Kanäle im Handstück und der Austrittsöffnung zu vermeiden. Die Filter können ebenfalls in einem in die Zuführungsleitungen oder zwischen diese und das Handstück einschaltbaren Zwischenstück gehaltert sein.

Vorzugsweise ist die Vorrichtung so ausgebildet, daß der Vorratsbehälter oder die Quelle für das zumindest eine Fluid mit einer Dosiervorrichtung für ein entkeimendes Fluid und/ oder mit einer anodischen Oxidationseinrichtung und/oder mit einer UV-Bestrahlungseinrichtung versehen ist und/oder daß der Vorratsbehälter für ein gasförmiges Fluid teilweise mit einer Flüssigkeit gefüllt ist, durch welches das gasförmige Fluid eingeleitet wird.

Es ist besonders praktisch, wenn ein zwischen Handstück und Quelle oder Vorratsbehälter einschaltbares, mit je einem Zulauf- und einem Ablauf für jedes Fluid versehenes Zwischenstück vorgesehen ist, in dem das zumindest eine Fluid UV-Strahlen ausgesetzt wird. Durch diese Maßnahme können auch bestehende Anordnungen in einfacher Weise mit einer Keimsperre versehen werden.

Weiter ist es zu bevorzugen, daß das zumindest eine Fluid im Bereich seiner Beaufschlagung mit UV-Strahlen in dafür durchlässigen Rohren, vorzugsweise Quarzrohren, geführt ist und daß die UV-Strahlen von dem UV-Strahler in vorzugsweise flexiblen Strahlungsleitern zu einem oder mehreren der verschiedenen Anwendungsstellen geführt sind. Duch diese letztere Maßnahme ergibt sich eine geringe Behinderung in der Bedienung der Handstücke infolge der Führung der Strahlungsleiter, und die Wartung wird vereinfacht.

Gemäß einer anderen Ausführungsform der Erfindung ist die Vorrichtung zum Sterilhalten zahnärztlicher oder chirurgischer Handstücke dadurch gekennzeichnet, daß der zumindest eine Flüssigkeits-Zuführungskanal zwischen Quelle oder Vorratsbehälter und den im Handstück befindlichen Austrittsöffnungen, vorzugsweise im Handstück, an eine Spannungsquelle anschließbare Elektroden enthält oder bildet, welche die Flüssigkeit während ihres Durchtretens entkeimen. Die Anode besteht dabei zweckmäßigerweise aus Silber. Es ist von Vorteil, wenn die eine Elektrode von zumindest Teilen der Innenwandung des Flüssigkeits-Zuführungskanals und die Gegenelektrode von einer im Inneren des Flüssigkeits-Zuführungskanals und gegenüber diesem isoliert gehalterten Elektrode gebildet werden. Die Elektrodenwirkung wird verbessert, wenn die Gegenelektrode parallel und vorzugsweise mittig zur Innenwandung des Flüssigkeits-Zuführungskanals verläuft und/oder in alternierenden Längsstücken isoliert und blank gelassen ist.

Andere Weiterbildungen der Erfindung sind in den Unteransprüchen angegeben und in den beigefügten Zeichnungen im einzelnen dargestellt.

### Beschreibung für den Vertragsstaat: AT

Die Erfindung betrifft eine Vorrichtung zum Sterilhalten der zahnärztlichen oder chirurgischen Handstücken zugeführten Fluide, aus denen die Fluide für Antriebs- und/oder Kühl- und/oder Spülzwecke, insbesondere Wasser und Druckluft, von einer Quelle oder einem Vorratsbehälter über Zuführungsleitungen zu Austrittsöffnungen in den Handstücken geführt sind.

An zahnärztlichen und chirurgischen Arbeitsplätzen werden verschiedene Handstücke verwendet, mit denen im Mund des Patienten oder in Wundhöhlen gearbeitet wird. Dies sind insbesondere sogenannte Luftturbinen, Handmotore, Spritz- bzw. Blashandstücke und sogenannte Zahnsteinentferner.

Die Luftturbinen werden nach dem Prinzip der Wasserräder, jedoch mit Druckluft, angetrieben. Beidseitig des Turbinenrades ist je ein Lager angebracht. Wegen der hohen Drehzahlen können diese Lager nicht abgedichtet werden.

Außerhalb der Lager, die sich im Kopfstück, im vordersten Teil des Bohrkopfes, befinden, sind Öffnungen vorhanden.

Durch die eine Öffnung ragt das Fräs- oder Schleifinstrument, die an der Gegenseite liegende Öffnung wird zum Wechseln des Fräs- oder Schleifinstrumentes benutzt. Durch diese Öffnungen gelangt Druckluft aus dem Bohrkopf in den Mund des Patienten. Ferner sind solche Luftturbinen mit Öffnungen am vordersten Teil des Bohrkopfes ausgestattet, aus denen Kühlwasser auf den Fräser oder Schleifer gespritzt wird. Dies ist wegen der hohen Drehzahlen und der damit verbundenen Erwärmung notwendig, um keine Schädigung am Zahn oder Knochen hervorzurufen.

Es gibt ferner mit Druckluft als auch elektrisch betriebene Handmotore, auf die Bohrköpfe aufsetzbar sind. Der Bohrer wird hier über Getriebe angetrieben. Auch diese Handstücke haben am Bohrkopf Kühlwasserausgänge zur Kühlung der Bohrer.

Spritz- und Blashandstücke haben die Aufgabe, flüssige Kühl- und/oder Spülfluide an die zu versorgende Stelle zu bringen oder Blasluft, z. B. zum Trockenblasen der Kavitäten.

Die Zahnsteinentfernungshandstücke haben ebenfalls Kühlwasserausgänge am vordersten Kopfteil.

Alle beschriebenen Handstücke werden über Zuführungsleitungen oder Schläuche mit den jeweiligen Fluiden z. B. Druckluft und Wasser versorgt. Diese Fluide sind normalerweise nicht keimfrei.

Zum anderen wird bei vielen dieser Handstücke ein Nachtropfen durch sogenannte Rücksaugsysteme verhindert, die automatisch beim Abschalten zumindest die Flüssigkeit unmittelbar vor und in den Leitungen im Bohrkopf in die Zuführungsleitungen oder Schläuche zurückziehen. Hierdurch werden Keime, die vom Mund des Patienten an die Handstücke gelangen, in diese eingesaugt.

Insbesondere das in den Zuführungsleitungen und den Vorratsbehältern in den Behandlungspausen stehende und meist zusätzlich noch erwärmte Fluid bildet einen fast idealen Nährboden für die Vermehrung der Keime, so daß es sogar vorgekommen ist, daß die Zuführungsleitungen von Kulturen der Keime soweit zugesetzt wurden, daß die Fluidzufuhr zu den Austrittsöffnungen eingeschränkt oder unterbrochen wurden.

An eine Sterilität im Wundbereich ist daher auch dann nicht mehr zu denken, wenn die Handstücke, soweit dies aufgrund ihrer Materialien möglich ist, von Zeit zu Zeit sterilisiert oder mit einer keimtötenden Flüssigkeit abgewischt werden. Hieraus resultiert eine unvermeidliche Infektionsgefahr im Wundgebiet.

Aus der GB-A-1 498 740 ist eine Vorrichtung zum Sterilhalten der zahnärztlichen oder chirurgischen Handstücken zugeführten Fluide für Antriebs- und/oder Kühl- und/oder Spülzwecke, insbesondere Wasser und Druckluft, von einer Quelle oder einem Vorratsbehälter über Zuführungsleitungen zu Austrittsöffnungen in den Handstücken geführt sind, bekannt, in der eine Metallionen emittierende Quelle, wie beispielsweise ein Kupferdraht, im Inneren der zu einem Handstück führenden Leitung vorgesehen ist. Die Verwendung einer solchen Quelle bewirkt nur dann eine ausreichende Keimabtötung, wenn die Konzentration der abgegebenen Metallionen sehr groß ist. Da das durch die Leitung geführte Spülwasser direkt in den Körper- und Wundbereich gerät, dürften bei ihm die Zugaben von derartigen Metallionen unter keinen Umständen höher sein als dies aus gesundheitlichen Gründen im Leitungswasser zulässig ist. Die danach zulässigen Dosierungen sind jedoch sehr gering, da die Metallionen in größerer Konzentration giftig sind. Aufgrund des intermittierenden Betriebs zahnärztlicher und chirurgischer Handstücke bleibt ein Teil der Flüssigkeit länger in der Leitung, so daß die Abgabe der Metallionen auf so geringe Konzentrationen eingestellt sein muß, daß auch in diesen Fällen - beispielsweise bei der Abschaltung über Nacht - die Dosierung nicht zu hoch wird. Dies bewirkt jedoch, daß bei einem kontinuierlichen Durchfluß von Spülflüssigkeit oder bei nur kurzfristig unterbrochenem Betrieb, bei dem jeweils Bakterien zurückgesaugt werden, die Abgabe der Metallionen nicht mehr ausreicht, um eine ausreichende Desinfektionswirkung zu erzielen.

Eine verbrauchsabhängige, genau dosierte Anreicherung der Spülflüssigkeit mit derartigen Metallionen in der zum Handstück geführten Leitung, in der pro Minute 0 bis 4.000 ml fließen, ist jedoch auf diesem Wege nicht möglich.

Dies bedeutet, daß im Falle eines hohen Durchflusses das aus dem Vorratsbehälter entnommene, im Normalfall nicht sterile Kühlfluid, wie beispielsweise Leitungswasser, nicht ausreichend entkeimt wird. Die Aussichten, daß aus dem Handstück zurückgesaugte Bakterien aufgrund der dann größeren Verweildauer in der Leitung abgetötet werden, sind zwar grösser. Wenn eine vollständige Entkeimung nicht stattfindet, können diese jedoch im Vorratsbehälter unter den dort für sie günstigen Temperaturbedingungen weiterwuchern, so daß es nach wie vor dazu kommen kann, daß die Behälter und Zuführungsleitungen ganz von Keimkulturen zugesetzt sind, bis die Fluidzufuhr zu den Austrittsöffnungen eingeschränkt oder unterbrochen wird.

Außerdem ist eine Vorrichtung der eingangs genannten Art in der nachveröffentlichten EP-A-773 beschrieben, die jedoch keine wirksame Keimabtötung im Rahmen der von den gesundheitsbehördlichen Vorschriften zugelassenen Toleranzen bei sehr stark variierendem Verbrauch an Spiel- und Kühlflüssigkeit ermöglicht.

Aufgabe der Erfindung ist es demgegenüber, eine Vorrichtung der eingangs genannten Art so auszubilden, daß sie eine wirksame Keimabtötung im Rahmen der nach den gesundheitsbehördlichen Vorschriften zulässigen Toleranzen für die beigegebenen Entkeimungsmittel ermöglicht, und zwar im Hinblick auf den bei zahnärztlichen und chirurgischen Behandlungselementen sehr stark variierenden Verbrauch an Spül- und Kühlflüssigkeit, wie er im Bereich von Nichtgebrauch der Vorrichtung über kurzzeitigen intermittierenden Betrieb bis zum kontinuierlichen Betrieb auftritt.

Diese Aufgabe wird erfindungsgemäß gelöst durch eine Dosiervorrichtung für ein zu entkeimendes Mittel mit einem ersten an eine Leitung über ein erstes Ventil anschließbaren Wasserbehälter, aus dem unten eine über ein zweites Ventil freigebbare Leitung herausführt und in den ein Injektor für die Freigabe einer dosierten Menge eines entkeimenden Mittels mündet, mit einem Schwimmer und einem Schwimmerventil, das mit den beiden Ventilen und dem Injektor derart zusammenwirkt, daß bei ausreichender Füllung des ersten Behälters das erste Ventil bei geschlossenem zweiten Ventil abschaltet und anschließend der Injektor erregt wird, und daß nach dem Entkeimen der im ersten Behälter befindlichen Wassermenge das zweite Ventil geöffnet wird, und mit einem zweiten Behälter, in den die Leitung mündet, an dem unten eine Leitung zu einem Verbraucher führt und der einen zweiten Schwimmer mit einem Schwimmerventil enthält, so daß bei vollem zweiten Behälter das entkeimte Verbrauchswasser über das Ventil mit definiertem Ausfluß abgegeben wird.

Auf diese Weise wird eine trotz stark wechselndem Verbrauch an Spül- und Kühlflüssigkeit ausreichende Entkeimung innerhalb der zulässigen Toleranzen erreicht.

Weiterbildungen der Erfindung sind in den Unteransprüchen angegeben und in den angefügten Zeichnungen im einzelnen dargestellt welche im zweiten Beschreibenungsteil näher erläutert werden.

**Beschreibung für die Vertragsstaaten: CH, FR, GB, LU, NL, SE**

Die beiliegende Zeichnung bevorzugter Ausführungsbeispiele dient der weiteren Erläuterung der Erfindung.

Fig. 1 zeigt in schematischer Darstellung einen Vertikalschnitt durch eine erste Ausführungsform einer Vorrichtung zum Sterilhalten chirurgischer oder zahnärztlicher Handstücke mit verschiedenen Varianten zur Keimabtötung verwendeter oxydierender Mittel.

Fig. 2 zeigt in schematischer Darstellung einen Vertikalschnitt durch eine Variante des Fluidvorrats- und Zuführungsteiles der in Fig. 1 gezeigten Vorrichtung.

Fig. 3 zeigt in schematischer Darstellung einen Vertakalschnitt durch eine Variante der in Fig. 1 gezeigten Vorrichtung bezüglich der Zuführung der Mikrowellenstrahlung zu den einzelnen Anwendungsgebieten.

Fig. 4 zeigt einen vertikalen Längsschnitt längs der Linie IV/IV von Fig. 3 durch ein zwischen Handstück und Zuführungsleitung einschaltbares als Mikrowellenquelle ausgebildetes Zwischenstück.

Fig. 5 zeigt einen Schnitt längs der Linie V/V durch das in Fig. 4 gezeigte Zwischenstück.

Fig. 6 zeigt eine weitere Variante eines für die Beaufschlagung des Fluid mit Mikrowellen vorgesehenen Zwischenstücks, das in das Fluid-Zuleitungssystem herkömmlicher Vorrichtungen einsetzbar ist.

Fig. 7 zeigt einen Längsschnitt durch das Zwischenstück der Fig. 6 längs der Linie VII/VII.

Fig. 8 zeigt einen Querschnitt durch das Zwischenstück der Fig. 6 längs der Linie VIII/VIII von Fig. 7.

Fig. 9 zeigt im Längsschnitt eine Teildarstellung von einem Fluidzuführungskanal, von dem Teile als Elektroden ausgebildet sind.

Fig. 10 zeigt eine Ansicht entsprechend zu derjenigen von Fig. 9, jedoch von einer Variante der Elektrodenanordnung.

Fig. 11 zeigt eine schematische Darstellung von einem Ausführungsbeispiel für die Entkeimung der Spülflüssigkeit.

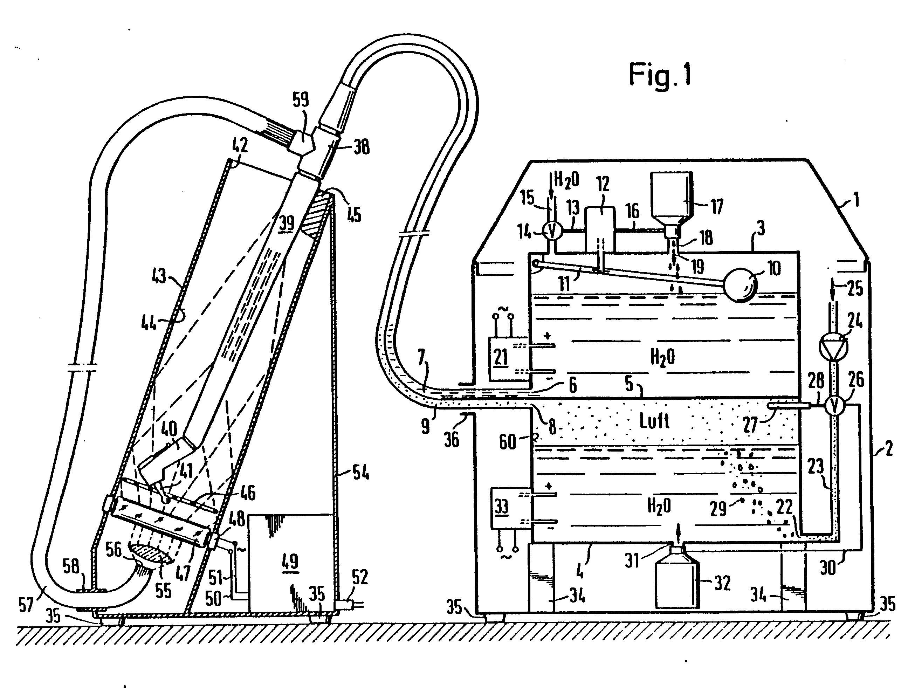

Die in Fig. 1 dargestellte Vorrichtung enthält in einem mit einem Deckel 1 versehen Gehäuse 2 einen Vorratsbehälter 3 für ein flüssiges Spülfluid, das beispielshalber mit Wasser angegeben ist, und einen Vorratsbehälter 4 für ein gasförmiges Fluid, das beispielsweise als Luft angegeben ist. Das Gehäuse 1 kann neben den im folgenden noch näher erläuterten Zusatzgeräten für die Aufbereitung, die Zuführung und die Weiterleitung der Fluide nicht näher dargestellte Antriebs-, Steuer- und Schaltmittel enthalten, wie sie zum Beispiel von Handstücken notwendig und üblich sind. Da diese jedoch nicht Gegenstand der Erfindung sind, wurden sie aus Übersichtlichkeitsgründen weggelassen. Die Vorratsbehälter 3 und 4 grenzen mittels einer gemeinsamen Trenwandung 5 aneinander an, wobei der Vorratsbehälter 3 für das Wasser oberhalb des Vorratsbehälters 4 für die Luft angebracht ist. Am in der Zeichnung unteren linken Ende des Vorratsbehälters 3 ist eine Austrittsöffnung 6 vorgesehen, an die eine Flüssigkeitsleitung 7 angeschlossen ist. An dem aus der Zeichnung linken oberen Ende des Vorratsbehälters 4 ist eine Austrittsöffnung 8 vorgesehen, an die eine Luftleitung 9 angeschlossen ist. Die Flüssigkeitsleitung 7 und die Luftleitung 9 sind, wie in der Zeichnung angedeutet, zu einem gemeinsamen flexiblen Zuführungsschlauch zusammengefaßt.

Der Vorratsbehälter 3 enthält des weiteren einen in die Oberfläche des Wassers eintauschenden Schwimmer 10, der mittels einer gelenkig angelenkten Stange 11 eine Steuervorrichtung 12 beträgt. Die Steuervorrichtung 12 regelt in Abhängigkeit von der Lage des Schwimmers 10 über ein Stellglied 13 ein Ventil 14, das in eine in den Behälter 3 oben mündende Anschlußleitung 15 für Frischwasser eingeschaltet ist. Die Steuervorrichtung 12 steht des weiteren über ein Stellglied 16 mit einer Oxydationsmitteldosiervorrichtung 17 in Verbindung, welche im Ansprechen auf das von der Steuervorrichtung 12 gelieferte Signal über eine oben in den Behälter 3 mündende Leitung 18, wie durch den Pfeil 19 angedeutet, dem im Behälter 3 befindlichen Wasser dosiert ein Oxydationsmittel, wie beispielsweise Chlor, kolloidales Silber u.ä. zuführt und damit die Entkeimung des Wassers bewirkt oder zu dieser beiträgt. Die Entkeimung des Wassers kann zusatzlich oder alternativ auch mittels einer lediglich schematisch angedeuteten anodischen Oxydationseinrichtung 21 bewirkt werden. Da derartige anodische Oxydationseinrichtungen bekannt sind, ist auf diese nicht näher eingegangen.

Der Behälter 4 ist in seinem unteren Bereich ebenfalls wie aus der Zeichnung ersichtlich, mit einer Flüssigkeit z. B. mit Wasser gefüllt. An seinem aus der Zeichnung rechten unteren Ende ist eine Zulauföffnung 22 vorgesehen, an der eine Druckluftleitung 23 angeschlossen ist. Die Druckluftleitung 23 führt zu einem Kompressor 24, der, wie durch den Pfeil 25 angedeutet, Luft ansaugt und verdichtet. In der Druckluftleitung 23 ist des weiteren ein Steuerventil 26 vorgesehen, das manuell oder von einem in den Luftraum des Behälters 4 ragenden Manometer 27 über ein Stellglied 28 steuerbar ist. Über das Manometer 27 und das Ventil 26 wird bei einem Abfall des Drucks in dem über dem Wasser befindlichen Luftraum des Behälters 4 unter einen gewünschten Wert weitere von dem Kompressor 24 zur Verfügung gestellte Druckluft durch das unten im Behälter 4 befindliche Wasser eingeleitet, die, wie durch das Bezugszeichen 29 angedeutet, in Form von Blasen nach oben steigt. Im Bereich der Zulauföffnung 22 kann eine nicht dargestellte Düse angebracht sein, welche zu einer Feinverteilung der eingeblasenen Druckluft führt. Von dem Steuerventil 26 zweigt des weitern eine Steuerleitung 30 ab, die ein Steuersignal einer am unteren Ende des Behälters 4 angebrachten, und mittels einer Austrittsöffnung 31 in diesen mündenden Oxydationsmitteldosiervorrichtung 32 zuführt. Im unteren, mit Wasser gefüllten Bereich des Vorratsbehälters 4 kann des weiteren, wie durch das Bezugszeichen 33 angedeutet, eine anodische Oxydationseinrichtung angebracht sein, welche zusammen mit der Oxydationsmitteldosiereinrichtung 32 oder anstelle dieser das in dem Vorratsbehälter befindliche Wasser und die durch diese entretende Druckluft entkeimt.

Mit 34 sind zwei Standfüsse für die Vorratsbehälter in dem Gehäuse 2, mit 35 Standfüsse des Gehäuses 2 und eines weiteren später noch näher erläuterten Gehäuses 54 und mit 36 eine Austrittsöffnung für die Leitungen 7 und 9 aus dem Gehäuse 2 angedeutet.

Die zu einem Schlauch zusammengefaßte Flüssigkeitsleitung 7 und die Luftleitung 9 führen über ein im folgenden noch näher erlautertes einschaltbares und eine Mikrowellenstrahlungsquelle bildendes Zwischenstück 38 zu einem Handstück 39, an dem ein Bohrkopf 40 mit einem Bohrer 41 gehaltert sind.

Das Handstück 39 ruht in einem an seiner Oberseite mit einer Öffnung 42 versehenen rohrartigen Köcher 43, der im Innener mit einer Verspiegelung 44 versehen ist. Mit 45 ist eine Halterung oder eine Auflage für das Handstück 39 angedeutet. Ein am unteren Ende des Köchers 43 angebrachtes Gitter 46 verhindert, daß das in den Köcher eingesetzte Handstück 39 an einem unterhalb des Gitters 46 in dem Köcher gehaltenen Mikrowellenstrahler 47 anstößt, der dort mittels eines Sockels 48 derart gelagert ist, daß seine Strahlung, von dem Gitter 46 nahezu ungehindert, in den Köcher 43 eintritt, und mehrfach an der Verspiegelung 44 reflektiert, das Handstück 39, den Bohrkopf 40 un den Bohrkopf 41 mit Mikrowellen bestrahlt, so daß etwaige außen an diesen oder an ihren Anstrittsöffnungen haltende Keime angetötet werden. Der Mikrowellenstrahler 47 ist an eine Spannungsversorgung und Steuereinrichtung 49, über Leitung 50, 51 angeschlossen. Mit 52 ist eine elektrische Zugführung zu der Spannungsversorgung 49 angedeutet. Der Köcher 43 und ein Gehäuse 54, welches die Spannungsversorgung 49 aufnimmt, sind im dargestellten Beispiel zu einer Einheit zusammengefaßt, welche, einzeln oder zusammen, wie in der Zeichnung nicht dargestellt, auch in das Gehäuse 2 integriert sein können.

Unterhalb des Mikrowellenstrahlers 47 ist, wie durch die Linse 55 angedeutet, eine Optik angebracht, welche die von dem Mikrowellenstrahler 47 austretende Strahlung auf eine Eintrittsende 56 eines flexiblen Lichtleiters 57 konzentriert. Der flexible Lichtleiter 57 tritt aus dem Gehäuse 54 über einen Austrittskanal 58 aus und führt zu einem Anschlußstück 59 des Zwischenstückes 38.

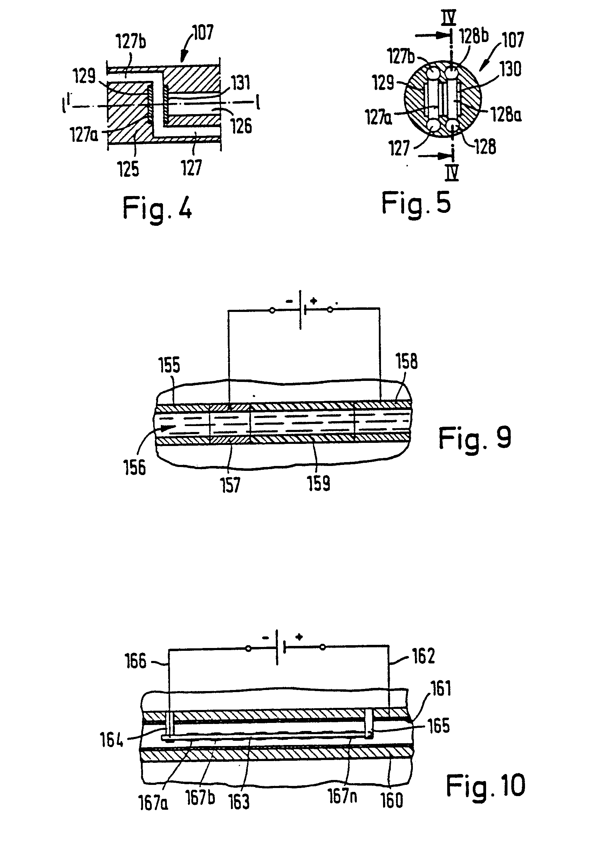

Bei dem Betrieb der Vorrichtung wird das in dem Behalter 3 befindliche Wasser durch die Zugabe eines Oxydationsmittels von der Oxydationsmitteldosiervorrichtung 17 und/oder durch die anodische Oxydationseinrichtung 21 entkeimt. Gleiches geschieht mit der Luft und dem Wasser in dem Vorratsbehälter 4 mittels der Oxydationsdosiervorrichtung 32 und/oder der anodischen Oxydationseinrichtung 33. Gleichzeitig wird das Handstück 39 im Köcher 43 von den von dem Mikrowellenstrahler 47 angehenden Mikrowellen außen sterilisiert. Über die Optik 55 und den flexiblen Lichtleiter 57 werden des weiteren Mikrowellen in das Zwischenstück 38 geleitet, in dem sie in ähnlicher Weise, wie in dem in den Figures 4 und 5 gezeigten Beispiel, eine Entkeimung der durchgeleiteten Fluide vornehmen, in dem sie die von den Vorratsbehältern 3 und 4 herführenden Leitungen 7 und 9 durchstrahlen, die im Bereich des Zwischenstücks 38 aus einem mikrowellendurchlässigen Material gefertigt sind. Wenn mit dem Handstück gearbeitet werden soll, wird es dem Köcher 43 entnommen, und an dem Arbeitsbereich gebracht. In herkömmlicher, nicht näher erläuterten Weise werden der Bohrer, die Spül- und Kühlwasserzufuhr und die Druckluftzufuhr betätigt, wobei das Wasser nach einem Abschalten jeweils in bekannter Weise wieder zurückgesaugt wird. Bei dem Durchtritt des Wassers und der Druckluft durch das Zwischenstück 38 werden diese entkeimt. Die den Vorratsbehältern 3 und 4 entnommenen Wasser- oder Luftmengen werden automatisch durch die Bauelemente 12 mit 15 bzw. 22 mit 28 ergänzt unter gleichzeitiger dosierter Zuführung einer entsprechenden Menge eines Oxydationsmittels, welches die notwendige Entkeimung sicherstellt. Die Zuführungsleitungen und die Kanäle im Inneren des Handstücks 39 sowie des Bohrkopfs 40 sowie auch die Innenwandungen der Vorratsbehälter 3 und 4 können durch das Bezugszeichen 60 angedeutet, mit einer Silberschicht oder einer Schicht eines entsprechend wirkenden Materials versehen sein, das dort den Ansatz von Keimen hindert.

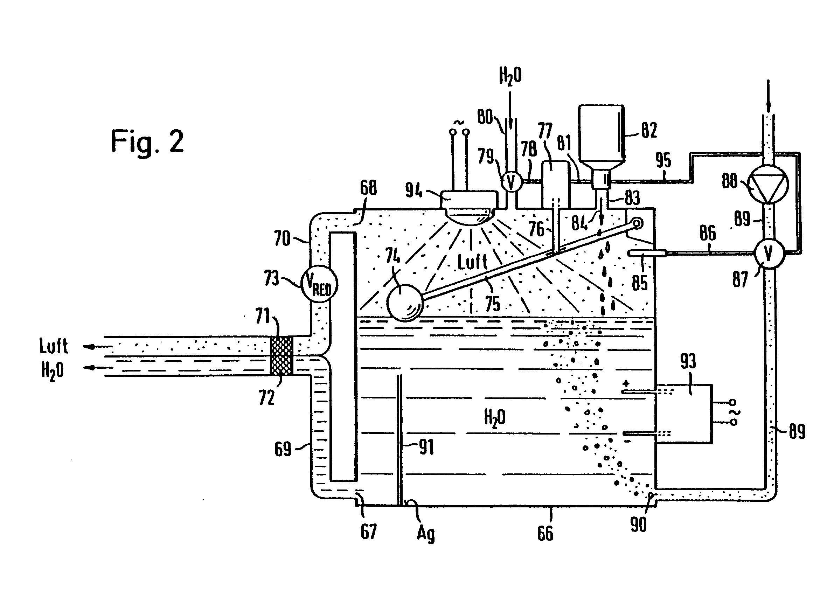

In Fig. 2 ist ein Ausführungsbeispiel dargestellt, bei dem für das gasförmige und für das flüssige Fluid lediglich ein gemeinsamer Vorratsbehälter 66 verwendet ist. Der Vorratsbehälter 66 enthält gemäß der Darstellung von Fig. 2 in seinem linken unteren Bereich eine Auslaßöffnung 67 für das unten im Behälter 66 befindliche Wasser und an seinem linken oberen Ende eine Auslaßöffnung 68 für Druckluft. An der Auslaßöffnung 67 ist eine Flüssigkeitsleitung 69, an der Auslaßöffnung 68 eine Druckluftleitung 70 angeschlossen. Die Leitungen 69 und 70 sind außerhalb des Behälters 2 zu einem gemeinsamen Schlauch zusammengefaßt. In der Flüssigkeitsleitung 69 und in der Druckluftleitung 70 sind Filter 71 und 72 angebracht. Die Druckluftleitung 70 enthält des weiteren ein steuerbares Reduzierventil 73. In die Oberfläche des Wassers taucht in entsprechender Weise wie in dem Vorratsbehälter 3 ein Schwimmer 74 ein, der mittels einer gelenkig befestigten Stange 75 und eines an dieser befestigten Stellgliedes 76 eine Steuervorrichtung 77 betätigt. Die Steuervorrichtung 77 steht über einem Stellglied 78 mit einem Ventil 79 in Wirkverbindung, das in eine oben in den Behälter 66 mündenden Zuführungsleitung 80 für das Wasser eingesetzt ist. Die Steuervorrichtung 77 steht des weiteren mittels eines Stellglieds 81 in Wirkverbindung mit einer Oxydationsmitteldosiervorrichtung 82, welche über eine oben in dem Behälter 66 mündende Leitung 83 wie durch den Pfeil 84 angedeutet beim Anheben des Schwimmers 74 nach erfolgter Absenkung eine Oxydationsmittelzugabe freisetzt. In den Luftraum des Behälters 66 mündet des weiteren ein Manometer 85, das mittels eines Stellglieds 86 in Wirkverbindung mit einem Ventil 87 steht, welches stromab von einem Kompressor 88 in einer Druckluftleitung 89 angebracht ist, die an dem aus der Sicht der Zeichnung rechten unteren Ende des Vorratsbehälters 66 in diesen über eine Einlaßöffnung 90 mündet. Im unteren von dem Wasser eingenommenen Bereich des Vorratsbehälters 66 ist eine nicht bis an die Oberfläche des Wasserspiegels reichende Trennwandung 91 angebracht, welche verhindert, daß die durch die Einlaßöffnung 90 eintretenden Luftblasen, bevor sie in den Luftraum gelangen, von der Flüssigkeitsleitung 69 über die Auslaßöffnung 67 abgesaugt werden. Die Entkeimung in dem Vorratsbehälter 66 kann zusätzlich oder unabhängig von der Oxydationsmittelzugabe über die Oxydationsmitteldosiervorrichtung 82 durch eine mit dem Wasser in Verbindung stehende anodische Oxydationseinrichtung 93 oder eine in der Zeichnung überhalb des Luftraums angedeuteten Mikrowellenstrahlungsquelle 94 vorgenommen werden.

Die Zugabe des Oxydationsmittels kann wie bereits vorstehend erläutert, im Ansprechen auf das Absinken und anschließende Anheben des Wasserspiegels und/oder wie durch eine von dem Ventil 87 zu der Oxydationsdosiermittelzugabe 82 verlaufende Steuerleitung 95 angedeutet, im Ansprechen auf einen von dem Manometer 85 ermittelten Druckabfall in dem über dem Wasser befindlichen Gasraum gesteuert werden. Der Betrieb der Vorrichtung von Fig. 2 entspricht im wesentlichen demjenigen des in Fig. 1 gezeigten Beispiels mit der Ausnahme, daß nunmehr Druckgas und Spülwasser in einem gemeinsamen Behälter sind, dort gemeinsam entkeimt werden, und unabhängig voneinander über die Offnungen 67 und 68 abgezogen werden.

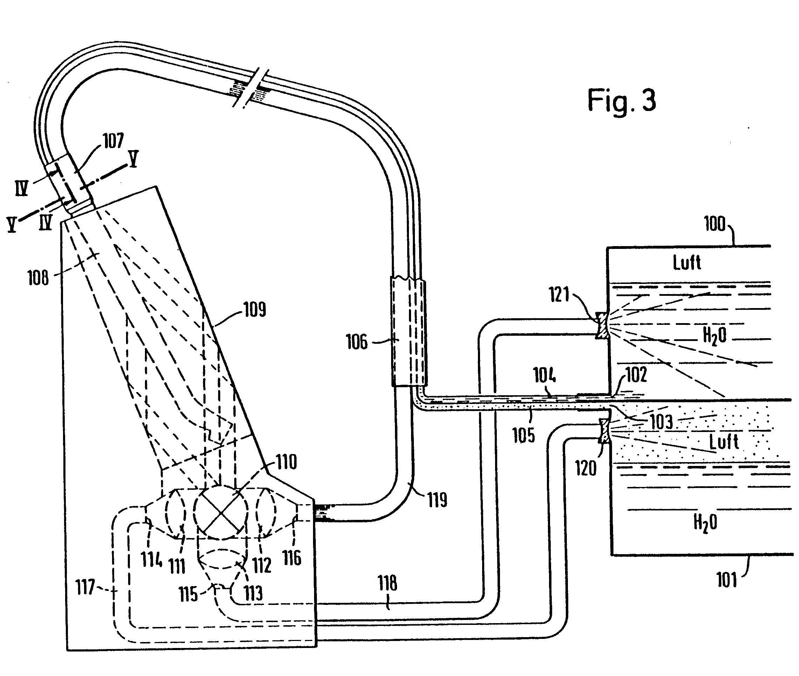

Bei der in Fig. 3 gezeigten Vorrichtung sind entsprechend wie bei dem Beispiel von Fig. 1 zwei getrennte und entsprechend ausgebildete Vorratsbehälter vorgesehen, ein Vorratsbehälter 100 für ein flüssiges Spüfluid, im dargestellten Falle Wasser, und ein Vorratsbehälter 101 für ein gasförmiges Fluid, im dargestellten Fall Druckluft. An dem unteren Ende des Vorratsbehälters 100 ist ein Auslaß 102 vorgesehen, an dem oberen Ende des Vorratsbehälters 101 für Druckluft ein Auslaß 103. An dem Auslaß 102 ist eine Flüssigkeitsleitung 104, an dem Auslaß 103 eine Druckluftleitung 105 angeschlossen, die in einem gemeinsamen flexiblen Schlauch 106 über ein Zwischenstück 107 zu einem lediglich strichliert angedeuteten Handstück 108 geführt sind. Das Handstück 108 steht in einem innen verspiegelten und mit Kurzwellen beaufschlagter Köcher 109 in entsprechender Weise wie bei dem in Fig. 1 gezeigten Beispiel, wobei die Mikrowellen von einem unterhalb des Köchers angebrachten Mikrowellenstrahler 110 geliefert werden. Dem Mikrowellenstrahler 110 gegenüberliegend sind Optiken 111, 112, und 113 angeordnet, welche die von dem Mikrowellenstrahler 110 ausgehenden Mikrowellen auf Eintrittsöffnungen 114, 115 und 116 von flexiblen Strahlungsleitern 117, 118 und 119 konzentrieren. Der Strahlungsleiter 117 führt zu einer zerstreuenden Optik 120, die im Bereich des Luftraumes des Vorratsbehälters 101 in dessen Wandung angebracht ist. Der Strahlungsleiter 118 führt zu einer entsprechenden zerstreuenden Optik 121, die in der Wandung des Vorratsbehälters 109 im Bereich des dort vom Wasser eingenommenen Raumes angebracht ist. Der Strahlungsleiter 119 ist schließlich in das Innere des die Flüssigkeitsleitung 104 und die Druckluftleitung 105, sowie gegebenenfalls eine elektrische Zuleitung enthaltenden flexiblen Schlauch 106 eingeführt, wo er mit diesem zu dem Zwischenstück 107 führt, in dem er wie näher in den Fig. 4 und 5 dargestellt endet.

Bei der in Fig. 3 dargestellten Ausführungsform werden somit mittels eines einzigen Mikrowellenstrahlers 110 direkt eine Entkeimung des Außenbereichs des Handstücks 108 und über die Strahlungsleiter 117, 118 und 119 eine Entkeimung der Druckluft in dem Vorratsbehälter 101 eine Entkeimung des Wassers in dem Vorratsbehälter 100 und eine Entkeimung des Wassers und der Druckluft in dem Zwischenstück 107 erreicht, wobei es für den letzteren Fall gleichgültig ist, ob diese Fluide von den Vorratsbehältern zu den Öffnungen in dem Handstück 108 oder umgekehrt unter der Wirkung einer nicht gezeigten Rücksaugvorrichtung strömen. Die Vorrichtung von Fig. 3 hat den weiteren Vorteil, daß mit dem Handstück 108 lediglich ein Zuleitungsschlauch verbunden ist, was die Handhabung des Handstückes 108 erleichtert.

Die Schnittdarstellungen der Fig. 4 und 5 zeigen Details des Zwischenstücks 107. In einem kreiszylindrischen, an seinen Ende mit nicht dargestellten Schnellverschlüssen versehenen Gehäuse 125, das im dargestellten Falle aus Massivmaterial besteht, mündet mittig und axial verlaufend das Ende 126 des Strahlungsleiters 119. Von mit der Flüssigkeitsleitung 104 bzw. der Druckluftleitung 105 verbundenen und in der Zeichnung nicht näher gezeigten Einlässen führen Kanäle 127 und 128 parallel zu dem Ende 126 des Strahlungsleiters 119 in das Gehäuse 125 hinein. Die Kanäle 127 und 128 sind in dem Gehäuse 125 anschließend derart abgeknickt, daß sie annähernd senkrecht zu dessen Längsachse 1/1 verlaufen, und dabei das Gehäuse quer in Stücken 127a und 128a durchsetzen, bevor sie anschliessend wieder, parallel zu den Kanälen 127 und 128 umgelenkt als Stücke 127b und 128b in Richtung auf das Handteil führen. In den Stücken 127a und 128a sind die Wandungen der Kanäle durch Quarzglasrohre 129 und 130 gebildet, an denen das freie Ende 131 des Strahlungsleiters stirnseitig anliegt. Durch diese Anordnung werden die durch die Leitungen 104, 127, 127a und 127b bzw. 105, 128, 128a und 128b strömenden Fluide einer starken Mikrowellenstrahlung im Bereich der Quarzglasrohre 129, 130 ausgesetzt, so daß dort die Keime agbetötet werden, wobei es gleichgültig ist, in welcher Richtung die Leitungen durchströmt sind.

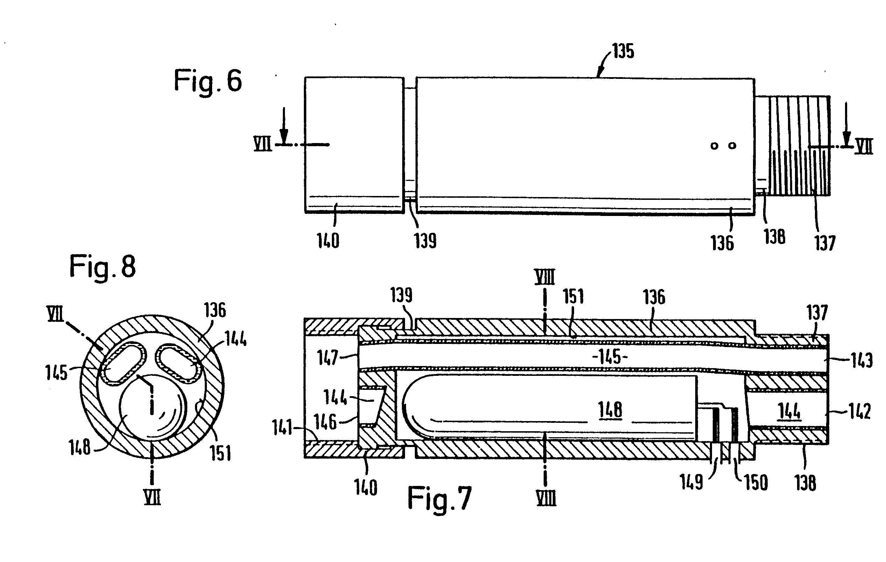

Die Fig. 6 mit 8 zeigen eine konkrete Ausführungsform eines Zwischenstücks, bei dem jedoch die Mikrowellen im Gegensatz zum vorstehenden Beispiel nicht mittels eines Strahlungsleiter in das Zwischenstück gebracht sondern dort mittels eines Mikrowellenstrahler erzeugt werden. Das Zwischenstück 135 besteht aus einer kreiszylindrischen Hülse 136, die vorzugsweise aus Metall besteht. Die Hülse 136 ist an einem Ende mit einem Ansatz 137 versehen, der einen geringeren Außendurchmesser aufweist als die Hülse und mit einem Außengewinde 138 versehen ist. Am anderen Ende der Hülse 136 ist in einer Nut 139 eine Überwurfmutter 140 lose drehbar aufgenommen, die ein Innengewinde 141 aufweist. Das zwischenstück 135 kann mittels seines Außengewindes 137 und einer der Überwurfmutter 140 entsprechenden, am Ende des die Druckluftleitung und die Spülfluidleitung enthaltenden Versorgungsschlauchs angebrachten Überwurmutter an das Ende des Versorgungsschlauchs angeschlossen werden. Durch geeignete in der Zeichnung nicht dargestellte Zentrier-, Ausricht- und Dichtungsmittel wird erreicht, daß das Ende der Druckluftleitung und das Ende der Spülfluidleitung einer Einlaßöffnung 142 für das Druckgas und einer Einlaßöffnung 143 für das Spülfluid so gegenüberliegen, daß eine dichte Verbindung hergestellt ist. Von den aus der Sicht der Fig. 6 und 7 an der rechten bzw. der rückwärtigen Stirnfläche des Zwischenstücks gelegenen Einlaßöffnungen 142 und 143 führen eine Druckluftleitung 144 und eine Spülfluidleitung 145 im Inneren durch die Hülse 136 und münden in der aus der Sicht der Fig. 6 und 7 linken, d.h. vorderen Stirnfläche derselben in Auslaßöffnungen 146 und 147. Die Einlaßöffnungen 142 und 143 sind bezogen auf eine senkrecht zur Längsachse des Zwischenstücks verlaufende Ebene spiegelbildlich zueinander angeordnet. Beim Befestigen des Zwischenstücks 135 mittels der Überwurfmutter 140 an dem Außengewinde eines Handstücks oder eines weiteren Versorgungsschlauches kommen daher die Austrittsöffnungen 146 und 147 den zugehörigen Einlaßöffnungen in den entsprechenden Bauelementen gegenüber zu liegen.

Im Inneren des Zwischenstücks ist ein sich nahezu über die gesamte Länge erstreckender Mikrowellenstrahler 148 angebracht, der mittels zweier aus der Hülse 136 herausführender Anschlüsse 149 und 150 an eine Spannungsquelle anschießbar ist. Die Druckluftleitung 144 und die Spülfluidleitung 145 sind im Inneren der Hülse 136, zumindest in dem der Mikrowellenstrahler gegenüberliegenden Bereich aus einem mikrowellen- bzw. UV-durchlössigen Material, wie Quarzglas gefertigt und derart angeordnet und geformt, daß sie dem Mikrowellenstrahler 148 möglichst große Oberfläche zukehren. Fig. 8 zeigt eine derartige Ausgestaltung, bei der die Druckluftleitung 144 und die Spülfluidleitung 145 abgeflacht sind, wobei die eine ihrer flachen Seiten dem Mikrowellenstrahler 148 zugekehrt ist.

Das Innere der Hülse 136 kann des weiteren, wie durch das Bezugszeichen 151 angedeutet, verspiegelt sein, so daß die aus dem Mikrowellenstrahler 148 austretenden Mikrowellen, sofern sie nicht direkt die in der Leitung 144 geführte Druckluft und das in der Leitung 145 geführte Spüfluid durchsetzen, dies nach Reflexionen an der Schicht 151 tun.

Das in den Fig. 6 mit 8 beschriebene Zwischenstück hat den Vorteil, daß es zwischen herkömmlichen Versorgungsschlauchstücken bzw. zwischen dem Versorgungsschlauch und die herkömmlicherweise verwendeten Handstücke eingeschaltet werden kann, ohne daß weitere bauliche Veränderungen notwendig sind.

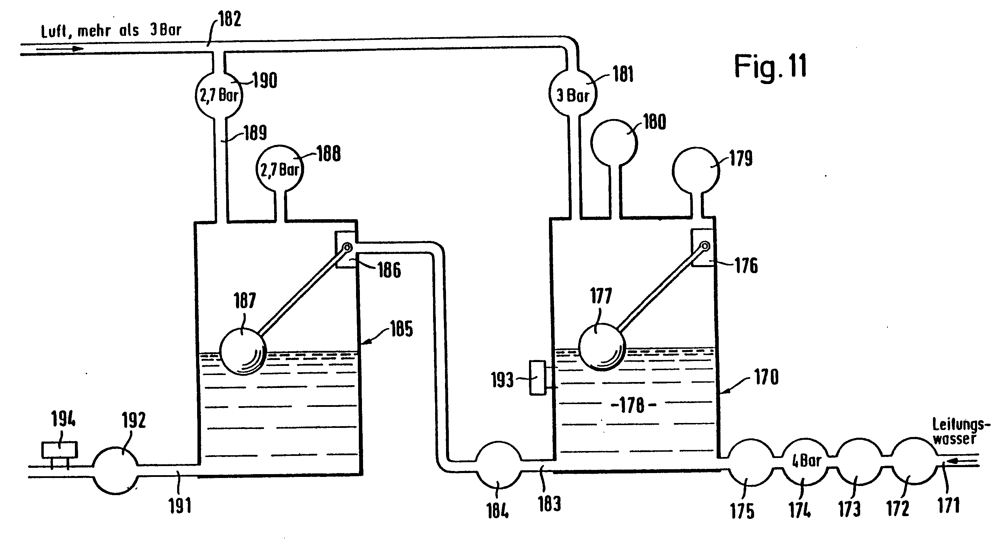

Fig. 9 zeigt in schematischer Darstellung die Ausgestaltung eines Spülfluidskanales 155 mit einer Elektrodenanordnung, mittels derer ein wie durch den Pfeil 156 angedeutetes flüssiges Spülfluid eine Entkeimung erfährt. Der Spülfluidkanal 155 ist in einem Bereich seiner Wandung als Elektrode 157 ausgebildet, die mit dem negativen Pol einer Spannungsquelle in Verbindung steht. Der positive Pol der Spannungsquelle ist mit einer weiteren von einem Teil der Spülfluidkanalwandlung gebildeten Elektrode 158 elektrisch leitend verbunden. Die Elektroden 157 und 158 sind mittels eines isolierenden Wandungsteils 159 des Spülfluidkanals 155 voneinander getrennt. Die Elektrode 158, welche die Anode bildet, besteht vorzugsweise aus Silber.

Eine weitere Variante dieser Elektrode ist in Fig. 10 gezeigt. Ein Spülfluidkanal 160 ist in seinem Inneren mit einer leitenden Metall-, beispielsweise einer Silberschicht 161 ausgekleidet, der an dem einen Pol, beispielsweise dem positiven Pol einer Spannungsquelle, wie durch die Leitung 162 angedeutet, angeschlossen ist. In der Mitte des Spülfluidkanals 160 ist eine stabförmige Elektrode 163 mittels isolierter Abstandhalter 164, 165 gehaltert. Im Inneren des isolierenden Abstandhalters 164 ist eine Leitung 166 hindurchgeführt, die zum einen mit der stabförmigen Elektrode, zum anderen mit dem anderen Pol, im dargestellten Beispiel, dem negativen Pol der Spannungsquelle verbunden ist. Die stabförmige Elektrode 163 ist durch isolierende Überzüge 167a - 167n, die beispielsweise von Lackschichten gebildet sind, in aufeinander folgende leitende und nicht leitende Bereiche unterteilt, wobei der Strom von der Elektrodenschicht 161 lediglich zu den leitenden, d.h. nicht mit einem isolierenden Überzug 167a... versehen Bereich der Elektrode 163 fließt.

Die Ausgestaltung der Spülfluidkänale mittels Elektrodenanordnungen gemäß Fig. 9 und Fig. 10 kann an jeder Stelle von den Quellen für die Spülfluide bis zu deren Austrittsöffnungen in den Handstücken erfolgen. Vorzugsweise sind die Elektrodenanordnungen jedoch in den Handstücken selbst und dort möglichst nahe an den Austrittsöffnungen oder in direkt hinter den Handstücken angebrachten Zwischenstücken vorgesehen, so daß die in die Handstücke zurückgesaugten Keime möglichst rasch abgetötet werden und möglichst keine kontaminierten Stellen im Inneren der Handstücke verbleiben.

Die in Fig. 11 gezeigte Dosiervorrichtung für die Zugabe eines Oxidationsmittels besteht aus einem Wasserbehälter 170, an dessem unteren Ende eine Leitung 171 mündet welche in Richtung auf den Behälter einen Vorfilter 172, der beispielsweise mit Aktivkohle gefüllt ist, ein Rückschlagventil 173, ein Druckminderventil 174 und ein Ein-Ausschaltventil 175 enthält. Im Inneren des Behälters ist ein Schwimmerventil 176 mit einem Schwimmer 177 angebracht, der in das Spülfluid, beispielsweise Wasser, 178 eintaucht. An der Oberseite des Behälters 170 befindet sich ein Injektor 179 für das Oxidationsmittel und ein Entlüftungsventil 180. In die Oberseite des Behälters wird über ein Druckminderventil 181 Druckluft eingeführt, die von einer Leitung 182 herangeführt wird. An der Unterseite des Behälters 170 führt eine Leitung 183 heraus, in die ein Ein-Ausschaltventil 184 eingebracht ist. Die Leitung 183 mündet an der Oberseite eines zweiten Behälters 185, der mit einem Schwimmerventil 186 und einem Schwimmer 187 versehen ist. Auch der Behälter 185 enthält ein Entlüftungsventil 188. An seiner Oberseite mündet des weiteren eine Abzweigungsleitung 189 von der Druckluftleitung 182, in der ein zweiter Druckminderer 190 eingesetzt ist. An der Unterseite des Behälters 185 ist eine Leitung 191 angeschlossen, die über eine Drossel 192 zu dem Verbraucher führt. Die Druckminderer 174, 181 und 190 sind in dieser Reihenfolge auf abnehmende Drücke eingestellt, wobei beispielsweise der Druckminderer 174 auf 4 Bar, der Druckminderer 181 auf 3 Bar und der Druckminderer 190 auf 2,7 Bar eingestellt sind.

Im folgenden wird die Wirkungsweise des Gerätes erläutert.

Über die Leitung 171 wird in dem Behälter 170 bei geöffnetem Ventil 175 Wasser mit einem Druck eingeführt, der durch den Druckminderer 174 eingestellt ist. Sobald eine ausreichende Menge in dem Behälter 170 enthalten ist, schaltet das Schwimmerventil 176, von dem Schwimmer 177 betätigt, das Ventil 175 ab. Das Ventil 184 ist zu diesem Zeitpunkt geschlossen. Die im Behälter 170 befindliche definierte Wassermenge wird anschließend von dem Injektor 179 mit einer definierten Menge eines Oxidationsmittels versetzt, wobei das beispielsweise ein Silbersalz sein kann, wie es von der Firma Katadyn unter der Bezeichnung "Micropur" vertrieben wird. Es ist jedoch auch möglich durch elektrolytische Abscheidung, beispielsweise mittels der Elektrodenanordnung 193, die im Behälter 170 befindliche Flüssigkeit mit einer zur Entkeimung notwendigen Silbermenge zu versehen was über einen Prozeßrechner und einen Operationsverstärker automatisch erfolgen Kann.

Sobald die im Behälter 170 befindliche Wassermenge entkeimt ist, wird das Ventil 184 geöffnet. Durch den von dem Druckminderer 181 eingestellten Überdruck im Behälter 170, der größer ist als der vom Druckminderer 190 eingestellte Überdruck im Behälter 185, wird das entkeimte Wasser 178 über die Leitung 183 in den Behälter 185 gedrückt. Sobald der zweite Behälter voll ist, kann aus diesem das Verbrauchswasser über das Reduzierventil 192 mit definiertem Ausfluß abgegeben werden. In der Leitung 191 stromab von der Drossel 192 kann des weiteren mittels der Elektrodenanordnung 194 eine elektrolytische Silberzugabe erfolgen, was bevorzugt automatisch über einen Prozeßrechner und einen Operationsverstärker geregelt wird. Der Injektor 179 kann auch für die Hinzugabe eines Entkalkungsmittels verwendet werden.

**Patentansprüche** für die
Vertragsstaaten: CH, FR, GB, LU, NL und SE

1. Vorrichtung zum Sterilhalten chirurgischer oder zahnärztlicher Handstücke, aus denen Fluide für Antriebs- und/oder Kühl- und/oder Spülzwecke in eine Körperhöhle des Patienten austreten, bei der das zumindest eine Fluid zwischen seiner Quelle oder seinem Vorratsbehälter und der Austrittsöffnung im Handstück ersten entkeimenden Mitteln ausgesetzt wird, welche als Keimsperre zwischen Austrittsöffnung und Quelle oder Vorratsbehälter wirken, und das zumindest eine Fluid im Bereich seiner Quelle oder seines Vorratsbehälters zweiten entkeimenden Mitteln ausgesetzt wird, wobei eine Dosiervorrichtung für ein entkeimendes Mittel vorgesehen ist, dadurch gekennzeichnet, daß die Dosiervorrichtung für ein zu entkeimendes Mittel einen ersten an eine Leitung (171) über ein erstes Ventil (175) anschließbaren Behälter (170), aus dem unten eine über ein zweites Ventil (184) freigebbare Leitung (183) herausführt und in den ein Injektor (179) für die Freigabe einer dosierten Menge eines entkeimenden Mittels mündet, mit einem Schwimmer (177) und einem Schwimmerventil (176), das mit den beiden Ventilen (175, 184) und dem Injektor (179) derart zusammenwirkt, daß bei ausreichender Füllung des ersten Behälters (170) das erste Ventil (175) bei geschlossenen zweiten Ventil (184) anschaltet und anschließend der Injektor (179) erregt wird, und daß nach dem Entkeimen der im ersten Behälter (170) befindlichen Wassermenge das zweite Ventil (184) geöffnet wird, und daß die Dosiervorrichtung einen zweiten Behälter (185) enthält, in den die Leitung (183) mündet, an dem unten eine Leitung (191) zu einem Verbraucher führt und der einen zweiten Schwimmer (187) mit einem Schwimmerventil (186) enthält, so daß bei vollem zweiten Behälter (185) des entkeimte Verbrauchswasser über das Ventil (192) mit definiertem Ausfluß abgegeben wird.

2. Vorrichtung nach Anspruch 1, dadurch gekennzeichnet, daß anstelle des Injektors (179) eine Elektrodenanordnung (193) vorgesehen ist, die elektrolytisch eine zur Entkeimung notwendige Silbermenge abscheidet.

3. Vorrichtung nach einem der Ansprüche 1 oder 2, dadurch gekennzeichnet, daß in den ersten und/oder zweiten Behälter (170, 185) eine mit einer Druckluftquelle verbundene Druckluftleitung (182, 189) mündet.

4. Vorrichtung nach Anspruch 3, dadurch gekennzeichnet, daß in die zu den Behältern (170, 185) führende Druckluftleitung (182, 189) ein Druckminderventil (181, 190) eingeschaltet ist, welches bewirkt, daß der erste Behälter (170) mit einem höheren Druck beaufschlagt ist als der zweite Behälter (155) und daß im oberen Bereich der Behälter (185, 170) ein Entlüftungsventil (180, 188) vorgesehen ist.

5. Vorrichtung nach einem der vorstehenden Ansprüche, dadurch gekennzeichnet, daß in der zum Verbraucher führenden Leitung (191) und/oder in der Zuführungsleitung (179) ein Druckminderventil (192, 174) vorgesehen ist.

6. Vorrichtung nach einem der vorstehenden Ansprüche, dadurch gekennzeichnet, daß der Vorratsbehälter (3, 4, 66, 100, 101) oder die Quelle für das zumindest eine Fluid mit einer Dosiervorrichtung (17, 32, 82) für ein entkeimendes Fluid und/oder mit einer anodischen Oxidationseinrichtung und/oder mit einer UV-Bestrahlungseinrichtung (94, 120, 121) versehen ist und/oder daß der Vorratsbehälter (4, 66, 101) für ein gasförmiges Fluid teilweise mit einer Flüssigkeit gefüllt ist, durch welches das gasförmige Fluid eingeleitet wird.

7. Vorrichtung nach Anspruch 6, dadurch gekennzeichnet, daß für das gasförmige und für das flüssige Fluid ein gemeinsamer Vorratsbehälter (66) vorgesehen ist, der je einen Auslaß (67, 68) für jedes der Fluide aufweist.

8. Vorrichtung nach Anspruch 6 oder 7, gekennzeichnet durch zumindest einen UV-strahler (47, 94, 110, 148), der so angeordnet ist, daß die von ihm ausgehenden UV-Strahlen das zumindest eine Fluid auf seinem Weg von der Quelle oder dem Vorratsbehälter (3, 4, 66) zu den Austrittsöffnungen durchsetzen und/oder daß der zumindest eine UV-Strahler das Innere des oder der Vorratsbehälter (66) für das zumindest eine Fluid mit UV-Strahlen bestrahlen.

9. Vorrichtung nach Anspruch 8, dadurch gekennzeichnet, daß das zumindest eine Fluid im Bereich seiner Beaufschlagung mit UV-Strahlen in dafür durchlässigen Rohren (144, 145), vorzugsweise Quarzrohren, geführt ist und daß die UV-Strahlen von dem UV-Strahler (110) in vorzugsweise flexiblen Strahlungsleitern (117, 118, 119) zu einem oder mehreren der verschiedenen Anwendungsstellen (100, 107, 101) geführt sind.

10. Vorrichtung nach Anspruch 9, gekennzeichnet durch ein zwischen Handstück (39, 108) und Quelle oder Vorratsbehälter (3, 4, 66, 100, 101) einschaltbares, mit je einem Zulauf (142, 143) und einem Ablauf (146, 147) für jedes Fluid versehenes Zwischenstück (38, 107, 135), in dem das zumindest eine Fluid den UV-Strahlen ausgesetzt wird.

11. Vorrichtung nach Anspruch 6 oder 7, dadurch gekennzeichnet, daß der zumindest eine Flüssigkeits-Zuführungskanal zwischen Quelle oder Vorratsbehälter und den Austrittsöffnungen im Handstücke, vorzugsweise im Handstück selbst an eine Spannungsquelle anschließbare Elektroden (157, 158, 161, 161) enthält oder bildet, welche die Flüssigkeit während ihres Durchtretens entkeimen.

12. Vorrichtung nach Anspruch 7, dadurch gekennzeichnet, daß die Elektrode (16) von zumindest Teilen der Innenwandung des Flüssigkeits-Zufuhrungskanals (160) und daß die Gegenelektrode (163) von einer im Innern des Flüssigkeits-Zuführungskanals und gegenüber diesem isoliert gehalterten Elektrode gebildet ist.

13. Vorrichtung nach Anspruch 12, dadurch gekennzeichnet, daß die Gegenelektrode parallel und vorzugsweise mittig zur Innenwandung des Flüssigkeits-Zuführungskanals verläuft und/oder in alternierenden Längsstücken isoliert (167 a...n) und blank gelassen ist (Fig. 10).

14. Vorrichtung nach einem der vorstehenden Ansprüche, gekennzeichnet durch zumindest ein austauschbares und vorzugsweise keimtötendes Filter (71, 72) in der zumindest einen zwischen dem Vorratsbehälter (66) und den Austrittsöffnungen im Handstück verlaufenden Zuführungsleitung (69, 70) für das zumindest eine Fluid.

15. Vorrichtung nach einem der vorstehenden Ansprüche, dadurch gekennzeichnet, daß die Fluid-Zuführungsleitungen zwischen der Quelle oder dem Vorratsbehälter und den Austrittsöffnungen im Handstück ganz oder zumindest stromab von den letzten keimtötenden Mitteln serilisierbar und/oder innen mit einer keimresistenten Oberfläche (161) versehen sind.

**Patentansprüche** für den Vertragsstaat: AT

1. Vorrichtung zum Sterilhalten der zahnärztlichen oder chirurgischen Handstücken zugeführten Fluide, aus denen die Fluide für Antriebs- und/oder Kühl- und/oder Spülzwecke, insbesondere Wasser und Druckluft, von einer Quelle oder einem Vorratsbehälter über Zuführungsleitungen zu Austrittsöffnungen in den Handstücken geführt sind, gekennzeichnet durch eine Dosiervorrichtung für ein zu entkeimendes Mittel mit einem ersten an eine Leitung (171) über ein erstes Ventil (175) anschließbaren Wasserbehälter (170), aus dem unten eine über ein zweites Ventil (184) freigebbare Leitung (183) herausführt und in den ein Injektor (179) für die Freigabe einer dosierten Menge eines entkeimenden Mittels mündet, mit einem Schwimmer (177) und einem Schwimmerventil (176), das mit den beiden Ventilen (175, 184) und dem Injektor (179) derart zusammenwirkt, daß bei ausreichender Füllung des ersten Behälters (170) das erste Ventil (175) bei geschlossenem zweiten Ventil (184) abschaltet und anschließend der Injektor (179) erregt wird, und daß nach dem Entkeimen der im ersten Behälter (170) befindlichen Wassermenge das zweite Ventil (184) geöffnet wird, und mit einem zweiten Behälter (185), in den die Leitung (183) mündet, an dem unten eine Leitung (191) zu einem Verbraucher führt und der einen zweiten Schwimmer (187) mit einem Schwimmerventil (186) enthält, so daß bei vollem zweiten Behälter (185) das entkeimte Verbrauchswasser über das Ventil (192) mit definiertem Ausfluß abgegeben wird.

2. Vorrichtung nach Anspruch 1, dadurch gekennzeichnet, daß anstelle des Injektors (179) eine Elektrodenanordnung (193) vorgesehen ist, die elektrolytisch eine zur Entkeimung notwendige Silbermenge abscheidet.

3. Vorrichtung nach einem der Ansprüche 1 oder 2, dadurch gekennzeichnet, daß in den ersten und/ oder zweiten Behälter (170, 185) eine mit einer Druckluftquelle verbundene Druckluftleitung (182, 189) mündet.

4. Vorrichtung nach Anspruch 3, dadurch gekennzeichnet, daß in die zu den Behältern (170, 185) führende Druckluftleitung (182, 189) ein Druckminderventil (181, 190) eingeschaltet ist und/oder daß im oberen Bereich der Behälter (185, 170) ein Entlüftungsventil (180, 188) vorgesehen ist.

5. Vorrichtung nach einem der Ansprüche 1 bis 4, dadurch gekennzeichnet, daß in der zum Verbraucher führenden Leitung (191) und/oder in der Zuführungsleitung (179) ein Druckminderventil (192, 174) vorgesehen ist.

**Claims** for the Contracting States: CH, FR, GB, LU, NL and SE

1. An apparatus for keeping sterile surgical or dental instruments from which fluids for driving and/or cooling and/or rinsing purposes issue into a cavity in the patient's body, in which at least one of the fluids is exposed between its source or its reservoir and the outlet opening in the instrument to first sterilizing means which act as a germ barrier between the outlet opening and the source or reservoir and, in the vicinity of its source or reservoir, to second sterilizing means, a metering unit being provided for a sterilizing agent, characterized in that the metering unit for a medium to be sterilized comprises a first container (170) which is connectible to a line (171) via a first valve (175) and from the lower end of which extends a line (183) releasable by a second valve (184) and into which opens an injector (179) for releasing a measured quantity of a sterilizing agent; a float (177) and a float valve (176) which cooperates with the two valves (175, 184) and the injector (179) in such a way that, if the first container (170) is filled to an adequate level, the first valve (175) is switched off with the second valve (184) closed and the injector (179) is subsequently energized and, after the quantity of water present in the first container (170) has been sterilized, the second valve (184) is opened, and in that the metering unit comprises a second container (185) into which opens the line (183), at the lower end of which a line (191) leads to a consumer and which comprises a second float (187) with a float valve (186) so that, when the second container (185) is full, the sterilized water intended for consumption is released at a certain rate through the valve (192).

2. An apparatus as claimed in Claim 1, characterized in that an electrode arrangement (193) is provided instead of the injector (179), electrolytically depositing silver in a quantity sufficient for sterilization.

3. An apparatus as claimed in Claim 1 or 2, characterized in that a compressed air line (182, 189) connected to a compressed air source opens into the first and/or second container (170, 185).

4. An apparatus as claimed in Claim 3, characterized in that a pressure-reducing valve (181, 190) is incorporated in the compressed air line (182, 189) leading to the containers (170, 185), its effect being that a higher pressure is applied to the first container (170) then to the second container (185), and in that a vent valve (180, 188) is provided in the upper region of the containers (185, 170).

5. An apparatus as claimed in any of the preceding Claims, characterized in that a pressure-reducing valve (192, 174) is provided in the line (191) leading to the consumer and/or in the feed line (179).

6. An apparatus as claimed in any of the preceding Claims, characterized in that the reservoir (3, 4, 66, 100, 101) or the source for at least one of the fluids is provided with a metering unit (17, 32, 82) for a sterilizing fluid and/or with an anodic oxidation unit and/or with a UV-irradiation unit (94, 120, 121) and/or in that the reservoir (4, 66, 101) for a gaseous fluid is partly filled with a liquid through which the gaseous fluid is introduced.

7. An apparatus as claimed in Claim 6, characterized in that a common resevoir (66) is provided for the gaseous and/or for the liquid fluid, comprising one outlet (67, 68) for each of the fluids.

8. An apparatus as claimed in Claim 6 or 7, characterized by at least one UV-lamp (47, 94, 110, 148) which is arranged in such a way that the UV-rays which it emits pass through the at least one fluid on its way from the source or the resevoir (3, 4, 66) to the outlet openings and/or in such a way that the at least one UV-lamp irradiates the interior of the reservoir(s) (66) for the at least one fluid with UV-rays.

9. An apparatus as claimed in Claim 8, characterized in that, in the region where it is exposed to UV-rays, the at least one fluid is guided through tubes (144, 145) permeable to UV-rays, preferably quartz tubes, and in that the UV-rays from the UV-lamp (110) are guided through preferably flexible radiation guides (117, 118, 119) to one or more of the various points of use (100, 107, 101).

10. An apparatus as claimed in Claim 9, characterized by an intermediate element (38, 107, 135) which is designed to be installed between the instrument (39, 108) and the source of reservoir (3, 4, 66, 100, 101), being provided with an inlet (142, 143) and an outlet (146, 147) for each fluid, and in which the at least one fluid is exposed to the UV-rays.

11. An apparatus as claimed in Claim 6 or 7, characterized in that the at least one liquid feed line between the source or reservoir and the outlet openings in the instrument, preferably in the instrument itself, contains or forms electrodes (157, 158, 161, 163) connectible to a voltage source which sterilize the liquid as it flows through.

12. An apparatus as claimed in Claim 7, characterized in that the electrode (16) is formed by at least parts of the inner wall of the liquid feed line (160) and in that the counter-electrode (163) is formed by an electrode mounted inside and insulated from the liquid feed line.

13. An apparatus as claimed in Claim 12, characterized in that the counter-electrode extends parallel and preferably centrally to the inner wall of the liquid feed line and/or is insulated (167 a...n) and left bare over alternate sections.

14. An apparatus as claimed in any of the preceding Claims, characterized by at least one replaceable and preferably germicidal filter (71, 72) in the at least one liquid feed line (69, 70) for the at least one fluid which extends between the reservoir (66) and the outlet openings in the instrument.

15. An apparatus as claimed in any of the preceding Claims, characterized in that the fluid feed lines between the source of the reservoir and the outlet openings in the instrument are sterilizable and/or provided internally with a germ-resistant surface (161) either over their entire length or at least downstream of the last sterilizing means.

**Claims** for the Contracting State: AT

1. An apparatus for keeping sterile the fluids delivered to dental or surgical instruments from which the fluids for driving and/or cooling and/or rinsing purposes, more especially water and compressed air, are guided from a source or reservoir via feed lines to outlet openings in the instruments, characterized by a metering unit for a medium to be sterilized comprising a first water container (170) which is connectible to a line (171) via a first valve (175) and from the lower end of which extends a line (183) releasable by a second valve (184) and into which opens an injector (179) for releasing a measured quantity of a sterilizing agent; a float (177) and a float valve (176) which co-operates with the two valves (175, 184) and the injector (179) in such a way that, if the first container (170) is filled to an adequate level, the first valve (175) is switched off with the second valve (184) closed and the injector (179) is subsequently energized and, after the quantity of water present in the first container (170) has been sterilized, the second valve (184) is opened, and further comprising a second container (185) into which opens the line (183), at the lower end of which a line (191) leads to a consumer and which comprises a second float (187) with a float valve (186) so that, when the second container (185) is full, the sterilized water intended for consumption is released at a certain rate through the valve (192).

2. An apparatus as claimed in claim 1, characterized in that an electrode arrangement (193) is provided instead of the injector (179), electrolytically depositing silver in a quantity sufficient for sterilization.

3. An apparatus as claimed in claim 1 or 2, characterized in that a compressed air line (182, 189) connected to a compressed air source opens into the first and/or second container (170, 185).

4. An apparatus as claimed in claim 3, characterized in that a pressure-reducing valve (181, 190) is incorporated in the compressed air line (182, 189) leading to the containers (170, 185) and/or in that a vent valve (180, 188) is provided in the upper region of the containers (185, 170).

5. An apparatus as claimed in any of the preceding claims, characterized in that a pressure-reducing valve (192, 174) is provided in the line (191) leading to the consumer and/or in the feed line (179).

**Revendications** pour les Etats Contractants: CH, FR, GB, LU, NL, SE

1. Dispositif pour maintenir stériles des instruments chirurgicaux ou dentaires desquels sortent des fluides à des fins d'entraînement et/ou de refroidissement et/ou de rinçage dans une cavité du corps humain d'un patient, dispositif dans lequel au moins un fluide est exposé dans l'instrument, à des premiers produits germicides entre sa source ou son récipient réservoir et l'ouverture de sortie, produits qui agissent en tant qu'écran aux germes entre l'ouverture de sortie et la source ou le récipient réservouir et qu'au moins un fluide est exposé au niveau de sa source ou de son réservoir à des deuxièmes produits germicides, un dispositif de dosage étant prévu pour un produit germicide, caractérisé en ce que le dispositif de dosage comporte un premier récipient (170) pour un produit à stériliser, raccordé à une conduite (171) par une première valve (175), récipient dont sort part le bas une conduite (183) pouvant être ouverte par une deuxième valve (184) et récipient dans lequel débouche un injecteur (179) pour libérer une quantité dosée d'un produit germicide, présentant un flotteur (177) et une valve à flotteur (176) qui coopère avec les deux valves (175, 184) et l'injecteur (179) de manière que, lors d'un remplissage suffisant du premier récipient (170), la première valve se ferme lorsque la deuxième valve (184) est fermée et qu'ensuite l'injecteur (179) est excité, et qu'après la stérilisation du volume d'eau se trouvant dans le premier récipient (170), la deuxième valve (184) est ouverte, et que le dispositif de dosage comporte un deuxième récipient (185) dans lequel débouche la conduite (183), duquel une conduite (191) conduit à un consommateur, et qui présente un deuxième flotteur (187) avec une valve à flotteur (186) de sorte que lorsque le deuxième récipient (185) est plein, l'eau consommée stérilisée peut être évacuée par la valve (192) avec un débit déterminé.

2. Dispositif selon la revendication 1, caractérisé en ce qu'un dispositif d'électrodes (193) est prévu à la place de l'injecteur (179), dispositif qui dépose de façon électrolytique une quantité d'argent nécessaire à la stérilisation.

3. Dispositif selon l'une des revendications 1 ou 2, caractérisé en ce qu'une conduite d'air comprimé (182, 189) reliée à une source à air comprimé débouche dans le premier et/ou le deuxième récipient (170, 185).

4. Dispositif selon la revendication 3, caractérisé en ce qu'une valve de détente (181, 190) est placée dans la conduite d'air comprimé (182, 189) conduisant aux récipients (170, 185), valve qui a pour effet de charger le premier récipient (170) avec une pression plus élevée que le deuxième récipient (185) et que, dans la partie supérieure des récipients (185, 170), est prévue une valve de dégagement d'air (180, 188).

5. Dispositif selon l'une des revendications précédentes, caractérisé en ce qu'une valve de détente (192, 174) est prévue dans la conduite (191) conduisant au consommateur et/ou dans la conduite d'amenée (179).

6. Dispositif selon l'une des revendications précédentes, caractérisé en ce que le récipient (3, 4, 66, 100, 101) ou la source pour au moins un fluide est muni d'un dispositif de dosage (17, 32, 82) pour un fluide stérilisant et/ou d'une installation d'oxydation anodique et/ou d'une installation de rayonnement UV (94, 120, 121) et/ou que le récipient réservoir (4, 66, 101) pour un fluide gazeux est rempli en partie par un liquide par lequel le fluide gazeux est amené.

7. Dispositif selon la revendication 6, caractérisé en ce qu'un récipient réservoir commun (66) est prévu pour le fluide gazeux et pour le fluide liquide, récipient qui présente une évacuation (67, 68) pour chaque fluide.

8. Dispositif selon la revendication 6 ou 7, caractérisé par au moins une source à rayons UV (47, 94, 110, 148) qui est prévue de telle manière que les rayons UV en provenant traversent au moins un fluide sur son parcours de la source ou du récipient réservoir (3, 4, 66) vers les ouvertures de sortie et/ou qu'au moins une source de rayons UV expose l'intérieur du ou des récipients réservoirs (66) aux rayons UV pour au moins un fluide.

9. Dispositif selon la revendication 8, caractérisé en ce qu'au moins un fluide est conduit par des tubes (144, 145) transparents aux rayons UV, de préférence des tubes en quartz, avec des rayons UV à la hauteur de sa charge et que les rayons UV sont conduits de la source à rayons UV (110), de préférence par des conducteurs à rayons flexibles (117, 118, 119) à un ou plusieurs endroits d'utilisation différents (100, 107, 101).

10. Dispositif selon la revendication 9, caractérisé par une pièce intercalée (38, 107, 135) placée entre l'instrument (39, 108) et la source ou le récipient réservoir (3, 4, 100, 101), présentant une arrivée (142, 143) et une évacuation (146, 147) pour chaque fluide en ce qu'au moins un fluide est exposé aux rayons UV.

11. Dispositif selon les revendications 6 ou 7, caractérisé en ce qu'au moins un canal d'amenée de liquide entre la source ou le récipient réservoir et les ouvertures de sortie dans l'instrument comporte ou forme des électrodes (157, 158, 161, 163), de préférence pouvant être raccordées à une source de tension dans l'instrument même, électrodes qui stérilisent le liquide pendant son passage.

12. Dispositif selon la revendication 7, caractérisé en ce que l'électrode (16) est formée au mins par des parties de la paroi interne du canal d'amenée de liquide (160) et quie la contre électrode (163) est formée par une électrode retenue à l'intérieur du canal d'amenée de liquide et isolée par rapport à ce dernier.

13. Dispositif selon la revendication 12, caractérisé en ce que la contre-électrode se présente parallèlement et de préférence au centre par rapport à la paroi intérieure du canal d'amenée de liquide et/ou qu'elle est laissée nue et isolée en sections longitudinales (167a...n) (Fig. 10).

14. Dispositif selon l'une des revendications précédentes, caractérisé par au moins un filtre (71, 72) échangeable et de préférence germicide dans au moins une conduite d'amenée (69, 70) pour au moins un fluide entre le récipient réservoir (66) et les ouvertures de sortie dans l'instrument.

15. Dispositif selon l'une des revendications précédentes, caractérisé en ce que les conduites d'amenée de fluide entre la source ou le récipient réservoir et les ouvertures de sortie dans l'instrument sont munies entièrement ou au moins en aval des derniers produits germicides d'une surface (161) stérilisable et/ou résistante aux germes à l'intérieur.

**Revendications** pour l'Etat Contractant: AT

1. Dispositif pour maintenir la stérilité des fluides appliqués à des pièces manuelles dentaires ou chirurgicales, parmi lesquels les fluides destinés à l'entraînement et/ou au refroidissement et/ou au lavage, en particulier de l'eau et de l'air comprimé, sont amenés d'une source ou d'un réservoir à des orifices de sortie situés dans les pièces manuelles par l'intermédiaire de conduites d'amenée, caractérisé par un dispositif de dosage pour un produit à stériliser, comportant un premier réservoir à eau (170) à raccorder à une conduite (171) par l'intermédiaire d'une première valve (175), duquel sort inférieurement une conduite (183) pouvant être débloquée par l'intermédiaire d'une seconde valve (184) et dans lequel débouche un injecteur (179) pour débiter une quantité dosée d'un produit de stérilisation, pourvu d'un flotteur (177) et d'une soupape à flotteur (176) coopérant avec les deux valves (175, 184) et l'injecteur (179) de telle façon que, lorsque le premier réservoir (170) est suffisamment rempli, la première valve (175) est mise hors fonction, la seconde valve (184) étant fermée à la suite de quoi l'injecteur (170) est excité, et qu'après la stérilisation de la quantité d'eau se trouvant dans le premier réservoir (170), la seconde valve (184) s'ouvre, et comportant un second réservoir (185) dans lequel débouche une conduite (183) sur laquelle, inférieurement, une conduite (191) aboutit à un appareil utilisateur et qui contient un second flotteur (187) comportant une soupape à flotteur (186), de sorte que, lorsque le second réservoir (185) est plein, l'eau d'utilisation stérilisée est débitée à un débit de sortie défini par l'intermédiaire de la valve (192).

2. Dispositif selon la revendication 1, caractérisé en ce qu'au lieu de l'injecteur (179), on prévoit un ensemble d'électrodes (193) qui sépare électrolytiquement une quantité d'argent nécessaire pour la stérilisation.

3. Dispositif selon la revendication 1 ou 2, caractérisé en ce qu'une conduite a air comprimé (182, 189) reliée à une source d'air comprimé débouche dans le premier réservoir et/ou le second réservoir (170, 185).

4. Dispositif selon la revendication 3, caractérisé en ce qu'une soupape de détente (181, 190) est montée dans la conduite à air comprimé (182, 189) aboutissant aux réservoirs (170, 185), et/ou en ce qu'on prévoit une soupape de dégagement d'air (180, 188) dans la zone supérieure des réservoirs (185, 170).

5. Dispositif selon l'une des revendications 1 à 4, caractérisé en ce qu'on prévoit dans la conduite (191) aboutissant à l'appareil utilisateur et/ou dans la conduite d'amenée (179) une soupape de détente (192, 174).

Fig.1

0019 622

Fig. 2

Fig. 3

0 019 622

5

Fig. 4

Fig. 5

Fig. 9

Fig. 10

Fig. 6

Fig. 7

Fig. 8

Fig.11